Europäisches Patentamt

European Patent Office

Office européen des brevets

(19)

(11) **EP 1 352 626 B1**

(12) **FASCICULE DE BREVET EUROPEEN**

(45) Date de publication et mention
de la délivrance du brevet:
**27.10.2004 Bulletin 2004/44**

(51) Int Cl.⁷: **A61K 7/00**

(21) Numéro de dépôt: **03290631.5**

(22) Date de dépôt: **13.03.2003**

(54) **Composition cosmétique sous forme d'émulsion multiple comprenant un agent tenseur**

Kosmetische Zusammensetzung in Form einer Mehrphasenemulsion enthaltend ein Tensormaterial

Cosmetic composition in the form of a multiple emulsion comprising a tensor agent

(84) Etats contractants désignés:
**AT BE BG CH CY CZ DE DK EE ES FI FR GB GR HU IE IT LI LU MC NL PT RO SE SI SK TR**

(30) Priorité: **12.04.2002 FR 0204609**

(43) Date de publication de la demande:
**15.10.2003 Bulletin 2003/42**

(73) Titulaire: **L'OREAL**
**75008 Paris (FR)**

(72) Inventeurs:
- **Cassin, Guillaume**
  **91140 Villebon sur Yvette (FR)**
- **Roger, Véronique**
  **92220 Bagneaux (FR)**
- **Chevalier, Veronique**
  **94440 Villecresnes (FR)**

(74) Mandataire: **Renard, Emmanuelle**
**L'OREAL - D.I.P.I.**
**25-29 Quai Aulagnier**
**92600 Asnières (FR)**

(56) Documents cités:
**WO-A-98/29091          FR-A- 2 659 551**
**FR-A- 2 783 419**

- **BODMEIER R. ET AL.: "Process and formulation variables in the preparation of wax microparticles by a melt dispersion technique. II. W/O/W multiple emulsion technique for water-soluble drugs." JOURNAL OF MICROENCAPSULATION, vol. 9, no. 1, 1992, pages 99-107, XP000248524**

EP 1 352 626 B1

EP 1 352 626 B1

**Description**

La présente invention se rapporte à une composition comprenant une émulsion multiple E/H/E comprenant une phase aqueuse interne, une phase huileuse et une phase aqueuse externe, ladite émulsion renfermant au moins un agent tenseur présent au moins dans la phase aqueuse interne de ladite émulsion. Elle se rapporte aussi à l'utilisation cosmétique de cette composition.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement, tels que les α-hydroxy-acides, les β-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés.

A cette fin, il a été proposé depuis quelques années des agents à effet tenseur qui lissent immédiatement après application les rides et ridules et contribuent à atténuer les marques de fatigue. Ces composés agissent en formant un film provoquant la rétraction du *stratum corneum*, la couche cornée superficielle de l'épiderme. Des exemples de tels agents tenseurs sont notamment des dispersions de polymères naturels (WO 98/29091) ou synthétiques, qu'il s'agisse de dispersions aqueuses (WO 98/29092) ou huileuses, en particulier de polymères en "étoile" (EP-1 043 345) ou de polymère siliconé greffé (EP-1 038 519). Il a également été suggéré d'utiliser certains composés inorganiques, tels que des silicates mixtes (EP-1 008 340) ou une dispersion aqueuse de particules colloïdales d'une charge minérale, pour obtenir des effets similaires.

Toutefois, ces agents tenseurs de l'art antérieur sont rarement formulés en présence d'huile, en particulier dans des émulsions H/E ou E/H, mais plus souvent sous forme de sérums, c'est-à-dire de compositions aqueuses gélifiées. Il est en effet généralement observé que l'efficacité de ces composés était grandement diminuée en présence d'huile.

Or, la présence d'une phase huileuse dans une composition cosmétique est souvent souhaitable, que ce soit pour conférer un caractère émollient à la composition ou pour véhiculer divers actifs topiques liposolubles, tels que les vitamines A et E, notamment, qui sont particulièrement utiles dans des compositions anti-rides.

Il reste donc le besoin de disposer d'une composition à effet tenseur, contenant une phase huileuse, qui soit stable dans le temps en vue d'une utilisation commerciale et qui offre des propriétés de tension de la peau suffisantes pour lisser visiblement les rides et ridules dès son application.

Ce besoin est satisfait selon la présente invention par une composition comprenant une émulsion multiple E/H/E comprenant une phase aqueuse interne, une phase huileuse et une phase aqueuse externe, ladite émulsion renfermant au moins un agent tenseur produisant à une concentration de 7% dans l'eau, une rétraction du stratum corneum isolé de plus de 1% à 30°C sous une humidité relative de 40 %, caractérisée en ce que ledit agent tenseur est présent au moins dans la phase aqueuse interne de ladite émulsion.

On entend par « agent tenseur » des composés susceptibles d'avoir un effet tenseur, c'est-à-dire pouvant tendre la peau et, par cet effet de tension, lisser la peau et y faire diminuer voire disparaître de façon immédiate les rides et les ridules.

Pour être efficaces, les agents tenseurs utilisés selon l'invention doivent produire à une concentration de 7% dans l'eau, une rétraction du stratum corneum isolé de plus de 1% à 30°C sous une humidité relative de 40 %. Cette valeur de rétraction est mesurée au Dermomètre, selon la méthode décrite dans l'Exemple 1 ci-après.

La composition selon l'invention est adaptée à une application topique sur la peau et comprend donc généralement un milieu physiologiquement acceptable, c'est-à-dire compatible avec la peau et/ou ses phanères.

La quantité d'agent tenseur présente dans la composition peut varier dans une large mesure en fonction de l'effet recherché. A titre d'exemple, l'agent tenseur peut représenter de 0,01 à 10% en poids, et de préférence de 0,1 à 7% en poids, par rapport au poids total de la composition.

Des exemples d'agents tenseurs convenant à une utilisation dans la présente invention comprennent : les polymères synthétiques, les polymères d'origine naturelle, les protéines et hydrolysats de protéines végétales ; les silicates mixtes ; les microparticules de cire ; et les particules colloïdales de charge inorganique.

Le polymère synthétique peut notamment être choisi parmi : les polymères et copolymères de polyuréthanne ; les polymères et copolymères acryliques ; les polymères d'acide isophtalique sulfoné ; et les polymères siliconés greffés.

Les copolymères de polyuréthanne, les copolymères acryliques et les autres polymères synthétiques selon l'invention peuvent notamment être choisis parmi les polycondensats, les polymères hybrides et les réseaux de polymères interpénétrés (IPNs).

Par "réseau de polymères interpénétrés" au sens de la présente invention, on entend un mélange de deux polymères enchevêtrés, obtenu par polymérisation et/ou réticulation simultanée de deux types de monomères, le mélange obtenu ayant une température de transition vitreuse unique.

Des exemples d'IPNs convenant à une mise en oeuvre dans la présente invention, ainsi que leur procédé de préparation, sont décrits dans les brevets US-6,139,322 et US-6,465,001, par exemple.

De préférence, l'IPN selon l'invention comprend au moins un polymère polyacrylique et, plus préférentiellement, il comprend en outre au moins un polyuréthane ou un copolymère de fluorure de vinylidène et d'hexafluoropropylène.

Selon une forme d'exécution préférée, l'IPN selon l'invention comprend un polymère polyuréthane et un polymère polyacrylique. De tels IPNs sont notamment ceux de la série Hybridur qui sont disponibles dans le commerce auprès de la société AIR PRODUCTS.

Un IPN particulièrement préféré se trouve sous la forme d'une dispersion aqueuse de particules ayant une taille moyenne, en poids, comprise entre 90 et 110 nm et une taille moyenne, en nombre, d'environ 80 nm. Cet IPN a de préférence une température de transition vitreuse, Tg, qui va d'environ -60°C à +100°C. Un IPN de ce type est notamment commercialisé par la société AIR PRODUCTS sous la dénomination commerciale Hybridur X-01602. Un autre IPN convenant à une utilisation dans la présente invention est référencé Hybridur X18693-21.

D'autres IPNs convenant à une mise en oeuvre dans la présente invention comprennent les IPNs constitués du mélange d'un polyuréthane avec un copolymère de fluorure de vinylidène et d'hexafluoropropylène. Ces IPNs peuvent notamment être préparés comme décrit dans le brevet US-5,349,003. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société ATOFINA.

Des exemples de polymères siliconés greffés sont indiqués dans la demande EP-1 038 519, qui est incorporée ici par référence. Un exemple préféré de polymère siliconé greffé est le polysilicone-8 (nom CTFA) qui est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle. Un polymère de ce type est notamment disponible sous la dénomination commerciale VS 80 (à 10% dans l'eau) ou LO 21 (sous forme pulvérulente) auprès de la société 3M. Il s'agit d'un copolymère de polydiméthylsiloxane à groupements propylthio, d'acrylate de méthyle, de méthacrylate de méthyle et d'acide méthacrylique.

Comme polymères d'origine naturelle, on peut citer les polyholosides, par exemple sous forme d'amidon. Il peut s'agir notamment d'amidon d'origine naturelle, issu de riz, de maïs, de pomme de terre, de manioc, de pois, de froment, d'avoine, etc... Un autre type de polyholoside est constitué par les carraghénanes, alginates, agars, gellanes, polymères cellulosiques et pectines.

Le polyholoside utilisé dans la composition selon l'invention se trouvera de préférence sous la forme d'un microgel ou dispersion aqueuse de microparticules de gel, lesdites particules ayant par exemple un diamètre moyen compris entre 0,5 et 100 µm et de préférence entre 5 et 50 µm.

Le polymère d'origine naturelle peut également être choisi parmi les latex constitués par la résine shellac, la gomme de sandaraque, les dammars, les élémis, les copals, les dérivés cellulosiques, et leurs mélanges.

Des exemples de protéines végétales et hydrolysats de protéines végétales utilisables comme agents tenseurs selon l'invention sont constitués des protéines et hydrolysats de protéines de maïs, de seigle, de froment, de sarrasin, de sésame, d'épautre, de pois, de fève, de lentille, de soja et de lupin.

Une autre classe d'agents tenseurs utilisables selon l'invention est constituée par les silicates mixtes. Par cette expression, on entend tous les silicates d'origine naturelle ou synthétique renfermant plusieurs types de cations choisis parmi les métaux alcalins (par exemple Na, Li, K) ou alcalino-terreux (par exemple Be, Mg, Ca) et les métaux de transition.

On utilise de préférence des phyllosilicates, à savoir des silicates ayant une structure dans laquelle les tétraèdres $SiO_4$ sont organisés en feuillets entre lesquels se trouvent enfermés les cations métalliques.

Une famille de silicates particulièrement préférée comme agents tenseurs est celle des laponites. Les laponites sont des silicates de magnésium, de lithium et de sodium ayant une structure en couches semblable à celle des montmorillonites. La laponite est la forme synthétique du minéral naturel appelé "hectorite". On peut utiliser par exemple la laponite commercialisée sous la dénomination Laponite XLS ou Laponite XLG par la société ROCKWOOD.

Une autre classe encore d'agents tenseurs utilisables dans la présente invention est constituée par les microparticules de cire. Il s'agit de particules ayant un diamètre généralement inférieur à 5 µm, ou mieux, à 0,5 µm, et constituées essentiellement d'une cire ou d'un mélange de cires choisies par exemple parmi les cires de Carnauba, de Candelila ou d'Alfa. Le point de fusion de la cire ou du mélange de cires est de préférence compris entre 50°C et 150°C.

En variante encore, on peut utiliser comme agent tenseur selon l'invention des particules colloïdales de charges inorganiques. Par "particules colloïdales", on entend des particules ayant un diamètre moyen en nombre compris entre 0,1 et 100 nm, de préférence entre 3 et 30 nm.

Des exemples de charges inorganiques comprennent : la silice, l'oxyde de cérium, l'oxyde de zirconium, l'alumine, le carbonate de calcium, le sulfate de baryum, le sulfate de calcium, l'oxyde de zinc et le dioxyde de titane. Une charge inorganique particulièrement préférée est la silice. Des particules colloïdales de silice sont notamment disponibles sous forme de dispersion aqueuse auprès de la société CATALYSTS & CHEMICALS sous les dénominations commerciales COSMO S-40 et COSMO S-50.

La composition selon l'invention peut être préparée selon toute méthode connue de l'homme du métier pour préparer des émulsions E/H/E et notamment par émulsification d'une émulsion inverse (E/H), dite émulsion primaire, dans une phase aqueuse gélifiée.

Il peut s'agir d'une émulsion triple eau/huile/eau, comportant une phase aqueuse externe gélifiée, une phase huileuse comprenant un émulsionnant siliconé, choisi en particulier parmi les alkyldiméthicone copolyols et les diméthicone copolyols, et éventuellement une huile de silicone, et une phase aqueuse externe contenant au moins un copolymère émulsionnant constitué d'une fraction majoritaire d'un monomère acide carboxylique monooléfiniquement insaturé en $C_3$-$C_6$ ou de son anhydride et d'une fraction minoritaire de monomère ester gras d'acide acrylique, en particulier un copolymère acrylate/$C_{10}$-$C_{30}$-alkylacrylate. Ce type d'émulsion triple est notamment décrit dans les demandes EP-0 908 170 et EP-0 648 102.

En variante, il peut s'agir d'une émulsion triple eau/huile/eau dont la phase aqueuse externe renferme un polymère ou copolymère d'acide acrylique ou méthacrylique associé à un polyglycéryl méthacrylate, et dont la phase huileuse peut renfermer une huile fluorée, comme décrit dans la demande EP-0 507 693.

En variante encore, il peut s'agir d'une émulsion eau/huile/eau dont l'une des phases aqueuses a une valeur d'activité en eau inférieure à 0,85, telle que décrite dans la demande EP-0 779 071.

Selon une forme d'exécution préférée de l'invention, la phase aqueuse externe est exempte de gélifiant à base de polyacrylamide, en particulier d'acide polyacrylamido méthylpropane sulfonique, et de triéthanolamine. Dans le cas notamment où l'agent tenseur est constitué de particules colloïdales de silice, la Demanderesse a en effet mis en évidence que la présence de l'un et/ou de l'autre de ces composés avait tendance à déstabiliser l'émulsion multiple E/H/E, conduisant à l'obtention d'une émulsion du type huile-dans-eau et à une prise en masse de la composition après un laps de temps de quelques heures à quelques jours. Une émulsion multiple E/H/E stable peut toutefois être obtenue en remplaçant la triéthanolamine par de l'hydroxyde de sodium, dans le cas où la présence d'un neutralisant est nécessaire dans la phase aqueuse externe, par exemple pour neutraliser un carbomer.

En outre, la Demanderesse a constaté que l'effet tenseur des compositions selon l'invention, en particulier lorsque l'agent tenseur est constitué de silice colloïdale, était amélioré en l'absence de polyol de faible poids moléculaire, tel que la glycérine. Ainsi, selon une autre forme d'exécution préférée de l'invention, la composition est exempte de glycérine et de propylène glycol.

La composition selon l'invention est notamment destinée à traiter les signes du vieillissement cutané et en en particulier à lisser ou atténuer les rides et ridules et/ou à retendre la peau.

L'invention a donc aussi pour objet un procédé de traitement cosmétique de la peau ridée, comprenant l'application sur la peau d'une composition telle que définie précédemment.

Elle a aussi pour objet l'utilisation cosmétique de la composition telle que définie précédemment pour lisser les rides et ridules et/ou retendre la peau, en particulier du visage et/ou du cou.

Pour renforcer les effets anti-âge de la composition selon l'invention, celle-ci peut renfermer, outre l'agent tenseur décrit précédemment, au moins un composé choisi parmi : les agents desquamants et/ou hydratants ; les agents dépigmentants ou propigmentants ; les agents anti-glycation ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents anti-pollution et/ou anti-radicalaire ; les agents amincissants ; les agents agissant sur la microcirculation ; les agents agissant sur le métabolisme énergétique des cellules ; et leurs mélanges.

Ainsi, la composition selon l'invention pourra notamment contenir au moins un actif choisi parmi : les α-hydroxyacides ; l'acide salicylique et ses dérivés tels que l'acide n-octanoyl-5-salicylique ; l'HEPES ; la procystéine ; l'O-octanoyl-6-D-maltose ; le sel disodique d'acide méthyl glycine diacétique ; les céramides ; les stéroïdes tels que la diosgénine et les dérivés de la DHEA ; l'acide kojique ; le N-éthyloxycarbonyl-4-para-aminophénol ; l'acide ascorbique et ses dérivés ; les extraits de myrtille ; les rétinoïdes et en particulier le rétinol et ses esters ; les polypeptides et leurs dérivés acylés ; les phytohormones ; les extraits de levure *Saccharomyces cerevisiae* ; les extraits d'algues ; les extraits de *Vitreoscilla filiformis* ; les extraits de soja, de lupin, de maïs et/ou de pois ; l'alvérine et ses sels, en particulier le citrate d'alvérine ; le resvératrol ; les caroténoïdes et en particulier le lycopène ; le tocophérol et ses esters ; le coenzyme Q10 ou ubiquinone ; les xanthines et en particulier la caféine et les extraits naturels en contenant ; les extraits de petit houx et de marron d'Inde ; et leurs mélanges, sans que cette liste soit limitative.

La composition selon l'invention peut en outre contenir au moins un filtre UVA et/ou UVB. Les filtres solaires peuvent être choisis parmi les filtres organiques, les filtres inorganiques et leurs mélanges.

Comme exemples de filtres organiques actifs dans l'UV-A et/ou l'UV-B, on peut citer notamment, désignés ci-dessous par leur nom CTFA :

- les dérivés de l'acide para-aminobenzoïque : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques : Homosalate vendu sous le nom « EUSOLEX HMS » par RONA/EM INDUSTRIES, Ethylhexyl Salicylate vendu sous le nom « NEO HELIOPAN OS » par HAARMANN et REIMER, Dipropyleneglycol Salicylate vendu sous le nom « DIPSAL » par SCHER, TEA Salicylate, vendu sous le nom « NEO HELIOPAN TS » par HAARMANN et REIMER,
- les dérivés du dibenzoylméthane : Butyl Methoxydibenzoylmethane vendu notamment sous le nom commercial « PARSOL 1789 » par HOFFMANN LA ROCHE, Isopropyl Dibenzoylmethane,
- les dérivés cinnamiques : Ethylhexyl Methoxycinnamate vendu notamment sous le nom commercial « PARSOL MCX » par HOFFMANN LA ROCHE, Isopropyl Methoxy cinnamate, Isoamyl Methoxy cinnamate vendu sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate : Octocrylene vendu notamment sous le nom commercial « UVINUL N539 » par BASF, Etocrylene, vendu notamment sous le nom commercial « UVINUL N35 » par BASF,
- les dérivés de la benzophénone : Benzophenone-1 vendu sous le nom commercial « UVINUL 400 » par BASF, Benzophenone-2 vendu sous le nom commercial « UVINUL D50 » par BASF, Benzophenone-3 ou Oxybenzone, vendu sous le nom commercial « UVINUL M40 » par BASF, Benzophenone-4 vendu sous le nom commercial « UVINUL MS40 » par BASF, Benzophenone-5, Benzophenone-6 vendu sous le nom commercial « HELISORB 11 » par NORQUAY, Benzophenone-8 vendu sous le nom commercial « SPECTRA-SORB UV-24 » PAR AMERICAN CYANAMID, Benzophenone-9 vendu sous le nom commercial« UVINUL DS-49» par BASF, Benzophenone-12,
- les dérivés du benzylidène camphre : 3-Benzylidene camphor, 4-Methylbenzylidene camphor vendu sous le nom « EUSOLEX 6300 » par MERCK, Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés du phényl benzimidazole : Phenylbenzimidazole Sulfonic Acid vendu notamment sous le nom commercial « EUSOLEX 232 » par MERCK, Benzimidazilate vendu sous le nom commercial commercial « NEO HELIOPAN AP » par HAARMANN et REIMER,
- les dérivés de la triazine : Anisotriazine vendu sous le nom commercial «TINOSORB S » par CIBA GEIGY, Ethylhexyl triazone vendu notamment sous le nom commercial «UVINUL T150 » par BASF, Diethylhexyl Butamido Triazone vendu sous le nom commercial « UVASORB HEB » par SIGMA 3V,
- les dérivés du phényl benzotriazole : Drometrizole Trisiloxane vendu sous le nom « SILATRIZOLE » par RHODIA CHIMIE ,
- les dérivés anthraniliques : Menthyl anthranilate vendu sous le nom commercial commercial « NEO HELIOPAN MA » par HAARMANN et REIMER,
- les dérivés d'imidazolines : Ethylhexyl Dimethoxybenzylidene Dioxoimidazoline Propionate,
- les dérivés du benzalmalonate : Polyorganosiloxane à fonctions benzalmalonate vendu sous la dénomination commerciale « PARSOL SLX » par HOFFMANN LA ROCHE,
- et leurs mélanges.

Les filtres UV organiques plus particulièrement préférés sont choisis parmi les composés suivants :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,

- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- et leurs mélanges.

Les filtres inorganiques utilisables dans la composition selon l'invention sont en particulier les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium. Des agents d'enrobage sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

La composition selon l'invention peut également contenir les adjuvants habituels dans les domaines cosmétique et dermatologique, tels que les gélifiants hydrophiles ou lipophiles, les conservateurs, les solvants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans l'une des phases aqueuses. Ces adjuvants, ainsi que leurs concentrations, doivent être tels qu'ils ne nuisent pas aux propriétés avantageuses de l'agent tenseur.

L'invention sera maintenant illustrée par les exemples non limitatifs suivants. Dans ces exemples, les quantités sont indiquées en pourcentage pondéral.

## EXEMPLES

EXEMPLES

### Exemple 1 : Composition cosmétique

| Émulsion primaire (A): | |
|---|---|
| Eau | 44,85 g |
| Polyglyceryl - 4 isostearate, hexyl laurate et cetyl PEG/PPG 10/1 dimethicone | 3,50 g |
| Cyclopentasiloxane | 16,50 g |
| Dimethicone | 4,00 g |
| Silice colloïdale | 31,15 g |

| Emulsion multiple | |
|---|---|
| Emulsion primaire A | 22,50 g |
| Cyclopentasiloxane | 3,50 g |
| Huile d'amande d'abricot | 4,00 g |
| Eau | 68,05 g |
| Conservateurs | 1,00 g |
| Pentasodium ethylene diamine tetramethylene phosphonate | 0,05 g |
| Copolymère alkylacrylate | 0,60 g |
| Hydroxyde de sodium | 0,30 g |

### Mode Opératoire

Préparation de l'émulsion primaire :

A température ambiante et sous agitation, on homogénéise le Polyglyceryl - 4 isostearate, l'hexyl laurate, le cetyl PEG/PPG 10/1 dimethicone, le cyclopentasiloxane et la dimethicone. Sous forte agitation, on incorpore lentement l'eau et la silice colloïdale.

Préparation de l'émulsion triple :

A température ambiante et sous agitation, on disperse le copolymère d'alkylacrylate, les conservateurs et le séquestrant (pentasodium éthylène diamine tetramethylene phosphonate). On laisse gonfler environ 45 minutes sous agitation puis on neutralise avec l'hydroxyde de sodium. On dilue l'émulsion primaire avec le cyclopentasiloxane et l'huile d'amande d'abricot puis on incorpore ce mélange lentement sous agitation à la phase aqueuse.

**Exemple comparatif 2 : Émulsion multiple E/H/E contenant un agent tenseur dans la phase aqueuse externe**

| Émulsion primaire (A): | |
|---|---|
| Eau | 75,20 g |
| Polyglyceryl - 4 isostearate, hexyl laurate et cetyl PEG/PPG 10/1 dimethicone | 3,50 g |
| Cyclopentasiloxane | 16,50 g |
| Dimethicone | 4,00 g |
| Sulfate de magnesium | 0,80 g |
| Emulsion multiple | |
| Emulsion primaire A | 22,50 g |
| Cyclopentasiloxane | 3,50 g |
| Huile d'amande d'abricot | 4,00 g |
| Eau | 81,05 g |
| Conservateurs | 1,00 g |
| Pentasodium ethylene diamine tetramethylene phosphonate | 0,05 g |
| Copolymère alkylacrylate | 0,60 g |
| Hydroxyde de sodium | 0,30 g |
| Silice colloïdale | 7,00 g |

**Exemples comparatifs 3 et 4 : Émulsions directe H/E et inverse E/H contenant un agent tenseur**

Le sens (E/H ou H/E) de l'émulsion est déterminé par l'ordre d'incorporation des phases aqueuse et huileuse. Ces émulsions sont préparées de manière classique pour l'homme du métier.

| Phase huileuse : | |
|---|---|
| Polyglyceryl - 4 isostearate, hexyl laurate et cetyl PEG/PPG 10/1 dimethicone | 0,79 g |
| Cyclopentasiloxane | 7,21 g |
| Dimethicone | 0,90 g |
| Huile d'amande d'abricot | 4,00 g |
| Phase aqueuse | |
| Eau | 78,15 g |
| Silice colloïdale | 7,00 g |
| Conservateurs | 1,00 g |
| Pentasodium ethylene diamine tetramethylene phosphonate | 0,05 g |
| Copolymère alkylacrylate | 0,80 g |
| Hydroxyde de sodium | 0,30 g |

**Exemple 5 : Emulsion triple E/H/E contenant un IPN**

| Émulsion multiple : | |
|---|---|
| Émulsion primaire (A) | 22,50 g |
| Cyclopentasiloxane | 3,50 g |
| Huile d'amande d'abricot | 4,00 g |
| Eau | 58,05 g |
| Phénoxyéthanol : | 1,00 g |
| Sel pentasodique d'éthylène diamine tétraméthylène phosphonate | 0,05g |
| Copolymère acide acrylique / méthacrylate de stéaryle (PEMULEN TR1 de NOVEON) | 0,60 g |
| Hydroxyde de sodium | 0,30 g |
| Dispersion aqueuse anionique à 40% d'un réseau interpénétré de polymères polyuréthanne et polyacrylique (HYBRIDUR 875 de AIR PRODUCTS) | 10,00 g |

| Émulsion primaire (A): | |
|---|---|
| Eau | 75,20 g |
| Mélange d'isostéaryle de polyglycéryle (4 moles), laurate d'hexyle et poly méthylcétyl diméthyl méthylsiloxane oxyéthyléné oxypropyléné (ABIL WE09 de GOLDSCHMIDT) | 3,50 g |
| Cyclopentasiloxane | 16,50 g |
| Polydiméthylsiloxane | 4,00 g |
| Sulfate de magnésium | 0,80g |

Mode Opératoire

*Préparation de l'émulsion primaire :*

A température ambiante et sous agitation, on homogénéise l'ABIL WE09, le cyclopentasiloxane et le polydiméthylsiloxane. Sous forte agitation, on incorpore lentement l'eau et le sulfate de magnésium.

*Préparation de l'émulsion multiple :*

A température ambiante et sous agitation, on disperse le copolymère acrylique, le phénoxyéthanol et le séquestrant phosphonique. On laisser gonfler environ 45 minutes sous agitation, puis on ajoute l'HYBRIDUR 875. On neutralise ensuite avec l'hydroxyde de sodium, puis on dilue l'émulsion primaire avec le cyclopentasiloxane et l'huile d'amande d'abricot. On incorpore alors ce mélange lentement sous agitation à la phase aqueuse.

**Exemple 6 : Mise en évidence de l'effet tenseur**

On a comparé dans cet exemple l'effet tenseur obtenu en utilisant les compositions des Exemples 1 à 4 ci-dessus, qui contiennent toutes 7% de silice colloïdale comme agent tenseur.

Ces compositions ont été testées au Dermomètre. Cet appareil a été décrit par L. Rasseneur et al. dans Influence des Différents Constituants de la Couche Cornée sur la Mesure de son Elasticité, International Journal of Cosmetic Science, 4, 247-260 (1982).

Le principe de la méthode consiste à mesurer la longueur d'une éprouvette de stratum corneum isolé à partir d'une peau humaine provenant d'une opération chirurgicale, avant et après traitement avec les compositions à tester.

Pour ce faire, l'éprouvette est placée entre les deux mâchoires de l'appareil, dont l'une est fixe et l'autre mobile, dans une atmosphère à 30°C et 40% d'humidité relative.

On exerce une traction sur l'éprouvette, et on enregistre la courbe de la force (en grammes) en fonction de la longueur (en millimètres), la longueur zéro correspondant au contact entre les deux mors de l'appareil.

On trace ensuite la tangente à la courbe dans sa région linéaire. L'intersection de cette tangente avec l'axe des abcisses correspond à la longueur apparente $L_o$ de l'éprouvette à force nulle.

On détend l'éprouvette puis on applique sur le stratum corneum 2 mg/cm$^2$ de la composition à tester. Après 15 mn de séchage, les étapes ci-dessus sont à nouveau mises en oeuvre pour déterminer la longueur $L_1$ de l'éprouvette après traitement.

Le pourcentage de rétraction est défini par :

$$\% \text{ rétraction} = 100 \times (L_1 - L_o)/L_o$$

Pour caractériser un effet tenseur, ce pourcentage doit être négatif et l'effet tenseur est d'autant plus important que la valeur absolue du pourcentage de rétraction est élevée.

Les résultats obtenus sont rassemblés dans le Tableau 1 ci-dessous :

Tableau 1

| Compositions | % de Rétraction d'une éprouvette de stratum corneum isolé |
|---|---|
| Composition témoin (Composition de l'Exemple 1 sans silice) | -0,23% +/- 0,20 |
| Exemple 1 : Émulsion multiple E/H/E à 7% de silice colloïdale en phase aqueuse interne | -1,18% +/- 0,34 |
| Exemple 2 : Émulsion multiple E/H/E à 7% de silice colloïdale en phase aqueuse externe | -0,69% +/- 0,29 |
| Exemple 3 : Émulsion directe H/E à 7% de silice colloïdale | -0,51% +/- 0,29 |
| Exemple 4 : Émulsion inverse E/H à 7% de silice colloïdale | -0,70% +/- 0,24 |

Il ressort du Tableau ci-dessus que seule la Composition de l'Exemple 1 (composition selon l'invention) présente une rétraction du stratum corneum supérieure, en valeur absolue, à 1%, lorsqu'elle est mesurée à 30°C et sous une humidité relative de 40%.

En particulier, la valeur de rétraction obtenue avec la Composition de l'Exemple 1 est significativement supérieure à celle obtenue avec la Composition de l'Exemple 2 (p<0,02).

Ainsi, la formulation d'un agent tenseur tel que la silice colloïdale dans d'autres systèmes galéniques (comprenant une phase huileuse) que la phase aqueuse interne d'une émulsion E/H/E ne permet pas d'obtenir un effet tenseur suffisant sur la peau.

**Exemple 7 : Evaluation sensorielle**

La composition de l'Exemple 1 a été appliquée sur un panel de sept femmes présentant des rides et ridules au niveau du contour de l'oeil. Il a été observé un effet de lissage des ridules se trouvant sous l'oeil après application de la composition.

**Revendications**

1. Composition comprenant une émulsion multiple E/H/E comprenant une phase aqueuse interne, une phase huileuse et une phase aqueuse externe, ladite émulsion renfermant au moins un agent tenseur produisant à une concentration de 7 % dans l'eau, une rétraction du stratum corneum isolé de plus de 1% à 30°C sous une humidité relative de 40 %, **caractérisée en ce que** ledit agent tenseur est présent au moins dans la phase aqueuse interne de ladite émulsion.

2. Composition selon la revendication 1, **caractérisée en ce que** ledit agent tenseur est choisi parmi : les polymères synthétiques, les polymères d'origine naturelle, les protéines et hydrolysats de protéines végétales ; les silicates mixtes ; les microparticules de cire ; et les particules colloïdales de charges inorganiques.

3. Composition selon la revendication 2, **caractérisée en ce que** ledit polymère synthétique est choisi parmi : les polymères et copolymères de polyuréthanne ; les polymères et copolymères acryliques ; les polymères d'acide isophtalique sulfoné ; et les polymères siliconés greffés.

4. Composition selon la revendication 2 ou 3, **caractérisée en ce que** ledit polymère synthétique est choisi parmi les réseaux de polymères interpénétrés.

5. Composition selon la revendication 4, **caractérisée en ce que** ledit polymère inerpénétré se trouve sous la forme d'une dispersion aqueuse de particules à base de polyuréthane et polyacrylique, ayant une taille moyenne, en poids, comprise entre 90 et 110 nm, une taille moyenne, en nombre, d'environ 80 nm et une température de transition vitreuse, Tg, qui va d'environ -60°C à +100°C.

6. Composition selon la revendication 3, **caractérisée en ce que** ledit polymère siliconé greffé est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle.

7. Composition selon la revendication 2, **caractérisée en ce que** ledit polymère d'origine naturelle est choisi parmi : les polyholosides et les latex.

8. Composition selon la revendication 7, **caractérisée en ce que** ledit polyholoside est un amidon choisi parmi : l'amidon de riz, de maïs, de pomme de terre, de manioc, de pois, de froment et d'avoine.

9. Composition selon la revendication 7, **caractérisée en ce que** ledit polyholoside est choisi parmi : les carraghénanes, alginates, agars, gellanes, polymères cellulosiques et pectines.

10. Composition selon l'une quelconque des revendications 7 à 9, **caractérisée en ce que** ledit polyholoside est sous forme de dispersion aqueuse de microparticules de gel.

11. Composition selon la revendication 7, **caractérisée en ce que** ledit latex est choisi parmi : la résine shellac, la gomme de sandaraque, les dammars, les élémis, les copals, les dérivés cellulosiques, et leurs mélanges.

12. Composition selon la revendication 2, **caractérisée en ce que** lesdits protéines et hydrolysats de protéines végétales sont choisis parmi : les protéines et hydrolysats de protéines de maïs, de seigle, de froment, de sarrasin, de sésame, d'épautre, de pois, de fève, de lentille, de soja et de lupin.

13. Composition selon la revendication 2, **caractérisée en ce que** ledit silicate mixte est une laponite.

14. Composition selon la revendication 2, **caractérisée en ce que** ladite cire est choisie parmi les cires de Carnauba, de Candelila et d'Alfa.

15. Composition selon la revendication 2, **caractérisée en ce que** ladite charge inorganique est choisie parmi : la silice, l'oxyde de cérium, l'oxyde de zirconium, l'alumine, le carbonate de calcium, le sulfate de baryum, le sulfate de calcium, l'oxyde de zinc et le dioxyde de titane.

16. Composition selon la revendication 15, **caractérisée en ce que** ladite charge inorganique est la silice.

17. Composition selon la revendication 2, 15 ou 16, **caractérisée en ce que** lesdites particules colloïdales ont un diamètre compris entre 3 et 30 nm.

18. Composition selon l'une quelconque des revendications 15 à 17, **caractérisée en ce que** la phase aqueuse externe est exempte de gélifiant à base de polyacrylamide et de triéthanolamine.

19. Composition selon l'une quelconque des revendication 1 à 18, **caractérisée en ce qu'**elle est adaptée à une application topique sur la peau.

20. Composition selon l'une quelconque des revendications 1 à 18, **caractérisée en ce que** ledit agent tenseur représente de 0,1 à 7% en poids, par rapport au poids total de la composition.

21. Composition selon l'une quelconque des revendications précédentes, **caractérisée en ce qu'**elle est exempte dé glycérine et de propylène glycol.

22. Procédé de traitement cosmétique de la peau ridée, comprenant l'application sur la peau de la composition selon l'une quelconque des revendications 1 à 21.

23. Utilisation cosmétique de la composition selon l'une quelconque des revendications 1 à 21 pour lisser les rides et ridules et/ou retendre la peau.

## Claims

1. Composition comprising a W/O/W multiple emulsion comprising an inner aqueous phase, an oily phase and an outer aqueous phase, the said emulsion containing at least one tensioning agent that produces, at a concentration of 7% in water, a retraction of isolated stratum corneum of more than 1% at 30°C under a relative humidity of 40%, **characterized in that** the said tensioning agent is present at least in the inner aqueous phase of the said emulsion.

2. Composition according to Claim 1, **characterized in that** the said tensioning agent is chosen from: synthetic polymers, polymers of natural origin, plant proteins and protein hydrolysates; mixed silicates; wax microparticles; and colloidal particles of mineral fillers.

3. Composition according to Claim 2, **characterized in that** the said synthetic polymer is chosen from: polyurethane polymers and copolymers; acrylic polymers and copolymers; sulphonated isophthalic acid polymers; and grafted silicone polymers.

4. Composition according to Claim 2 or 3, **characterized in that** the said synthetic polymer is chosen from networks of interpenetrated polymers.

5. Composition according to Claim 4, **characterized in that** the said interpenetrated polymer is in the form of an aqueous dispersion of particles based on polyurethane and polyacrylic, with a weight-average size of between 90 and 110 nm, a number-average size of about 80 nm and a glass transition temperature, Tg, which ranges from about -60°C to +100°C.

6. Composition according to Claim 3, **characterized in that** the said grafted silicone polymer is a polydimethylsiloxane onto which are grafted, via a linking chain of thiopropylene type, mixed polymer units of the poly(meth)acrylic acid type and of the polyalkyl (meth)acrylate type.

7. Composition according to Claim 2, **characterized in that** the said polymer of natural origin is chosen from: polyholosides and latices.

8. Composition according to Claim 7, **characterized in that** the said polyholoside is a starch chosen from: rice starch, corn starch, potato starch, cassava starch, pea starch, wheat starch and oat starch.

9. Composition according to Claim 7, **characterized in that** the said polyholoside is chosen from: carrageenans, alginates, agars, gellans, cellulose-based polymers and pectins.

10. Composition according to any one of Claims 7 to 9, **characterized in that** the said polyholoside is in the form of an aqueous dispersion of gel microparticles.

11. Composition according to Claim 7, **characterized in that** the said latex is chosen from: shellac resin, sandarac gum, dammar resins, elemi gums, copal resins and cellulose-based derivatives, and mixtures thereof.

12. Composition according to Claim 2, **characterized in that** the said plant proteins and protein hydrolysates are chosen from: proteins and protein hydrolysates from corn, rye, wheat, buckwheat, sesame, spelt, pea, bean, lentil, soybean and lupin.

13. Composition according to Claim 2, **characterized in that** the said mixed silicate is a laponite.

14. Composition according to Claim 2, **characterized in that** the said wax is chosen from carnauba wax, candelilla wax and alfalfa wax.

15. Composition according to Claim 2, **characterized in that** the said mineral filler is chosen from: silica, cerium oxide, zirconium oxide, alumina, calcium carbonate, barium sulphate, calcium sulphate, zinc oxide and titanium dioxide.

16. Composition according to Claim 15, **characterized in that** the said mineral filler is silica.

17. Composition according to Claim 2, 15 or 16, **characterized in that** the said colloidal particles have a diameter of between 3 and 30 nm.

18. Composition according to any one of Claims 15 to 17, **characterized in that** the outer aqueous phase is free of gelling agent based on polyacrylamide and on triethanolamine.

19. Composition according to any one of Claims 1 to 18, **characterized in that** it is suitable for topical application to the skin.

20. Composition according to any one of Claims 1 to 18, **characterized in that** the said tensioning agent represents from 0.1% to 7% by weight relative to the total weight of the composition.

21. Composition according to any one of the preceding claims, **characterized in that** it is free of glycerol and of propylene glycol.

22. Cosmetic process for treating wrinkled skin, comprising the application to the skin of the composition according to any one of Claims 1 to 21.

23. Cosmetic use of the composition according to any one of Claims 1 to 21, for smoothing out wrinkles and fine lines and/or for restoring tautness to the skin.


**Patentansprüche**

1. Zusammensetzung, die eine multiple W/O/W-Emulsion enthält, welche eine innere wässerige Phase, eine Ölphase und eine äußere wässerige Phase aufweist, wobei die Emulsion mindestens ein Straffungsmittel enthält, das in einer Konzentration von 7 % in Wasser bei 30 °C und unter einer relativen Feuchte von 40 % zu einer Retraktion von isoliertem *Stratum corneum* von mehr als 1 % führt, **dadurch gekennzeichnet, dass** das Straffungsmittel zumindest in der inneren wässerigen Phase der Emulsion vorliegt.

2. Zusammensetzung nach Anspruch 1, **dadurch gekennzeichnet, dass** das Straffungsmittel ausgewählt ist unter: synthetischen Polymeren, Polymeren natürlicher Herkunft, pflanzlichen Proteinen und pflanzlichen Proteinhydrolysaten; gemischten Silicaten; Wachsmikropartikeln; und kolloidalen Partikeln von anorganischen Füllstoffen.

3. Zusammensetzung nach Anspruch 2, **dadurch gekennzeichnet, dass** das synthetische Polymer ausgewählt ist unter: Polymeren und Copolymeren von Polyurethan, Acrylpolymeren und Acrylcopolymeren; Polymeren von sulfonierter Isophthalsäure; und gepfropften Siliconpolymeren.

4. Zusammensetzung nach Anspruch 2 oder 3, **dadurch gekennzeichnet, dass** das synthetische Polymer unter den Netzwerken von interpenetrierenden Polymeren ausgewählt ist.

5. Zusammensetzung nach Anspruch 4, **dadurch gekennzeichnet, dass** das interpenetrierende Polymer in Form einer wässerigen Dispersion von Partikeln auf der Basis von Polyurethan und Polyacrylverbindungen vorliegt, wobei es eine auf das Gewicht bezogene mittlere Größe von 90 bis 110 nm, eine auf die Anzahl bezogene mittlere Größe von etwa 80 nm und eine Glasübergangstemperatur Tg von etwa -60 °C bis +100 °C besitzt.

6. Zusammensetzung nach Anspruch 3, **dadurch gekennzeichnet, dass** das gepfropfte Siliconpolymer ein Polydimethylsiloxan ist, auf das über eine Verbindungsgruppe vom Thiopropylentyp gemischte Polymereinheiten vom Typ Poly(meth)acrylsäure und vom Typ Polyalkyl(meth)acrylat gepfropft wurden.

7. Zusammensetzung nach Anspruch 2, **dadurch gekennzeichnet, dass** das Polymer natürlicher Herkunft unter den Polyholosiden und Latices ausgewählt ist.

8. Zusammensetzung nach Anspruch 7, **dadurch gekennzeichnet, dass** das Polyholosid eine Stärke ist, die ausgewählt ist unter: Stärke aus Reis, Mais, Kartoffel, Maniok, Erbse, Weizen und Hafer.

9. Zusammensetzung nach Anspruch 7, **dadurch gekennzeichnet, dass** das Polyholosid ausgewählt ist unter: Carrageenanen, Alginaten, Agar-Agar, Gellanen, Cellulosepolymeren und Pectinen.

10. Zusammensetzung nach einem der Ansprüche 7 bis 9, **dadurch gekennzeichnet, dass** das Polyholosid als wässerige Dispersion von Gelmikropartikeln vorliegt.

11. Zusammensetzung nach Anspruch 7, **dadurch gekennzeichnet, dass** der Latex ausgewählt ist unter: Schellack, Sandarack, Dammar, Elemi, Kopalen, Cellulosederivaten und deren Gemischen.

12. Zusammensetzung nach Anspruch 2, **dadurch gekennzeichnet, dass** die pflanzlichen Proteine und Proteinhydrolysate ausgewählt sind unter: Proteinen und Proteinhydrolysaten von Mais, Roggen, Weizen, Buchweizen, Sesam, Einkorn, Erbse, Bohne, Linse, Soja und Lupine.

13. Zusammensetzung nach Anspruch 2, **dadurch gekennzeichnet, dass** das gemischte Silicat ein Laponit ist.

14. Zusammensetzung nach Anspruch 2, **dadurch gekennzeichnet, dass** das Wachs unter Carnaubawachs, Candelillawachs oder Alfawachs ausgewählt ist.

15. Zusammensetzung nach Anspruch 2, **dadurch gekennzeichnet, dass** der anorganische Füllstoff ausgewählt ist unter: Kieselsäure, Ceroxid, Zirconiumoxid, Aluminiumoxid, Calciumcarbonat, Bariumsulfat, Calciumsulfat, Zinkoxid und Titandioxid.

16. Zusammensetzung nach Anspruch 15, **dadurch gekennzeichnet, dass** der anorganische Füllstoff Kieselsäure ist.

17. Zusammensetzung nach Anspruch 2, 15 oder 16, **dadurch gekennzeichnet, dass** die kolloidalen Partikel einen Durchmesser von 3 bis 30 nm aufweisen.

18. Zusammensetzung nach einem der Ansprüche 15 bis 17, **dadurch gekennzeichnet, dass** die äußere wässerige Phase keinen Gelbildner auf der Basis von Polyacrylamid und Triethanolamin enthält.

19. Zusammensetzung nach einem der Ansprüche 1 bis 18, **dadurch gekennzeichnet, dass** sie für eine topische Anwendung auf die Haut geeignet ist.

20. Zusammensetzung nach einem der Ansprüche 1 bis 18, **dadurch gekennzeichnet, dass** das Straffungsmittel 0,1 bis 7 Gew.-%, bezogen auf das Gesamtgewicht der Zusammensetzung, ausmacht.

21. Zusammensetzung nach einem der vorhergehenden Ansprüche, **dadurch gekennzeichnet, dass** sie kein Glycerin und kein Propylenglykol enthält.

22. Verfahren zur kosmetischen Behandlung von Haut, die Falten aufweist, das das Aufbringen der Zusammensetzung nach einem der Ansprüche 1 bis 21 auf die Haut umfasst.

23. Kosmetische Verwendung der Zusammensetzung nach einem der Ansprüche 1 bis 21 zur Glättung von Falten und Fältchen und/oder zur Straffung der Haut.